# EUROPEAN PATENT APPLICATION

(11) **EP 4 657 724 A1**
(43) Date of publication of application: **03.12.2025**
(21) Application number: 24795921.6
(22) Date of filing: 17.04.2024
(51) Int. Cl.: H02K 11/30, H02K 11/20, A61M 60/122, A61M 60/216, A61M 60/422

(54) **ELECTRIC MOTOR AND VENTRICULAR ASSIST DEVICE**

(30) Priority: 27.04.2023 CN 202310481556
(71) Applicant: Shenzhen Core Medical Technology Co., Ltd., Shenzhen, Guangdong 518000 (CN)
(72) Inventor: XIAO, Zhenzhong, Shenzhen, Guangdong 518000 (CN); XIE, Duanqing, Shenzhen, Guangdong 518000 (CN); YU, Shunzhou, Shenzhen, Guangdong 518000 (CN)
(74) Representative: Appleyard Lees IP LLP
(86) International application number: PCT/CN2024/088202
(87) International publication number: WO 2024/222540

(57) **Abstract**

An electric motor (11) and a ventricular assist device (10). The electric motor (11) comprises a housing (200), a sensing assembly (300), a stator assembly (400) and a control assembly (500), wherein an accommodating cavity (210) is formed by means of enclosure of the housing (200); the sensing assembly (300) comprises a sensing circuit board (310), which is accommodated in the accommodating cavity (210); the stator assembly (400) penetrates the sensing circuit board (310); and the control assembly (500) comprises a control circuit board (520) electrically connected to the sensing circuit board (310), wherein the control circuit board (520) is accommodated in the accommodating cavity (210) and is arranged at the peripheral side of the stator assembly (400), such that the control circuit board (520) does not occupy a space of the electric motor (11) in an axial direction, thereby improving the safety performance of the ventricular assist device (10).

## Description

This application claims priority to Chinese patent application No. 202310481556.6, filed on April 27, 2023, the content of which is hereby incorporated by reference in its entirety.

### TECHNICAL FIELD

The present application relates to the technical field of medical devices, and in particular, to a motor and a ventricular assist device.

### BACKGROUND

The incidence of cardiovascular diseases is increasing annually, and patients with critical conditions such as cardiogenic shock and heart failure face an extremely high mortality rate. A ventricular assist device is a device capable of treating these diseases. Blood is propelled by the impeller of the ventricular assist device and then flows out, enabling the blood pumped by the ventricular assist device to meet the patient's requirements for blood flow and perfusion pressure. However, conventional ventricular assist devices often pose a risk of contact with other organs in the thoracic cavity, thereby affecting the safety of the ventricular assist device's use.

### SUMMARY

Accordingly, the present application provides a motor and a ventricular assist device with improved safety performance. The embodiments of the present application achieve the above objective through the following technical solutions.

Embodiments in a first aspect of the present application provide a motor, including:
a housing defining an accommodating cavity;
a sensing assembly including a sensing circuit board accommodated in the accommodating cavity;
a stator assembly extending through the sensing circuit board; and
a control assembly including a control circuit board electrically connected to the sensing circuit board, wherein the control circuit board is accommodated in the accommodating cavity and is provided at a peripheral side of the stator assembly.

Embodiments in a second aspect of the present application provide a ventricular assist device including a motor. The motor includes:
a housing defining an accommodating cavity;
a sensing assembly including a sensing circuit board accommodated in the accommodating cavity;
a stator assembly extending through the sensing circuit board; and
a control assembly including a control circuit board electrically connected to the sensing circuit board, wherein the control circuit board is accommodated in the accommodating cavity and is provided at a peripheral side of the stator assembly.

Details of one or more embodiments of the present application are set forth in the accompanying drawings and description below. Other features, objects, and advantages of the present application will become apparent from the description, drawings, and claims.

### BRIEF DESCRIPTION OF THE DRAWINGS

To illustrate the technical solutions according to the embodiments of the present application or in the prior art more clearly, the accompanying drawings for describing the embodiments or the prior art are introduced briefly in the following. Apparently, the accompanying drawings in the following description are only some embodiments of the present invention, and persons of ordinary skill in the art can derive other drawings from the accompanying drawings without creative efforts.
FIG. 1 is a schematic structural view of a ventricular assist device according to the present application.
FIG. 2 is a partially exploded view of the ventricular assist device shown in FIG. 1.
FIG. 3 is a schematic structural view of a motor according to the present application, with a housing removed.
FIG. 4 is a longitudinal sectional view of the motor of the ventricular assist device shown in FIG. 1.
FIG. 5 is a schematic structural view of a motor according to the present application.
FIG. 6 is a top view of the motor shown in FIG. 5.
FIG. 7 is a top view of a sensing assembly of the motor shown in FIG. 5.
FIG. 8 is a partial top view of the motor shown in FIG. 5.
FIG. 9 is a schematic structural view of the motor shown in FIG. 3 from another perspective.
FIG. 10 is a schematic structural view of a control assembly of the motor shown in FIG. 5.
FIG. 11 is a schematic structural view of a fixing member of the motor shown in FIG. 5.
FIG. 12 is a schematic structural view of the fixing member shown in FIG. 11 from another perspective.

### DETAILED DESCRIPTION

The present application will now be described in detail with reference to the accompanying drawings and embodiments in order to make the objectives, technical solutions, and advantages of the present application more clear. It should be understood that the specific embodiments described herein are only for explaining the present application, and not intended to limit the present application.

It should be noted that when an element is referred to as being "fixed to" or "arranged on" another element, it may be directly or indirectly disposed on the other element. When an element is referred to as being "connected to" another element, it may be directly or indirectly connected to the other element.

In addition, the terms "first" and "second" are used for descriptive purposes only, and cannot be construed as indicating or implying a relative importance, or implicitly specifying the number of the indicated technical features. Thus, the quantity of the feature defined with "first" or "second" may explicitly or implicitly be one or more. In the description of the present application, "a plurality of" means two or more, unless otherwise defined explicitly and specifically.

The inventors of the present application have found that the motor of the conventional ventricular assist device typically includes one sensing circuit board and one control circuit board. The sensing circuit board is provided adjacent to an end of the stator assembly in an axial direction thereof, and the control circuit board is provided adjacent to the other end of the stator assembly in the axial direction thereof. Consequently, the motor has a relatively large thickness in the axial direction. When considering the control circuit board, the stator assembly, and the sensing circuit board as a whole, the overall thickness is relatively large, leading to an increased thickness of both the motor and the ventricular assist device containing the motor. When a thick ventricular assist device is implanted into a patient's body, it increases the risk of contact with other organs in the thoracic cavity, thereby compromising the safety of the ventricular assist device's use.

It should be noted that in the context, the axial direction of the stator assembly and the axial direction of the motor have the same meaning, that is, the axial direction of the motor refers to the axial direction of the stator assembly. Similarly, the radial direction of the stator assembly and the radial direction of the magnetic core share the same meaning.

To address these issues, the present application provides a motor and a ventricular assist device. By providing the control circuit board of the motor on the peripheral side of the stator assembly, the control circuit board does not occupy space in the axial direction of the motor, thereby reducing the thickness of the motor, decreasing the risk of contact with other organs in the thoracic cavity when the ventricular assist device is implanted into a patient, and ultimately improving the safety performance of the ventricular assist device. The motor and the ventricular assist device provided in the present application will be illustrated in detail below with reference to specific embodiments and the accompanying drawings.

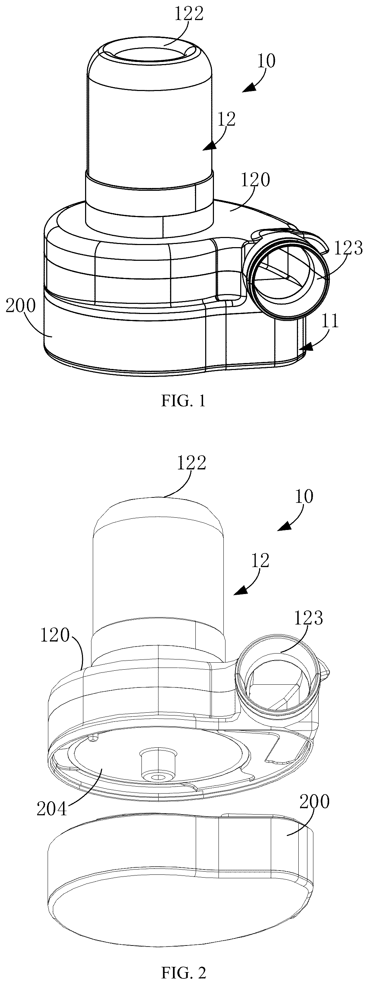

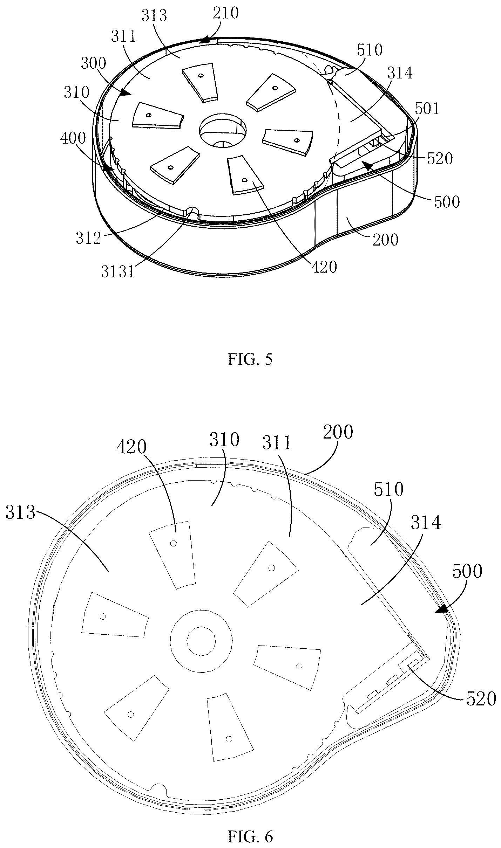

Referring to FIGS. 1 to 3, the ventricular assist device 10 provided in the present application includes a motor 11 and a pump body 12. The motor 11 includes a housing 200, a sensing assembly 300, a stator assembly 400, a control assembly 500 (see FIG. 5), and a rotor 600. The pump body 12 includes a pump case 120 and an impeller (not shown). The pump case 120 is detachably or non-detachably connected to the housing 200. The pump case 120 covers an opening of an accommodating cavity 210. The pump case 120 encloses and defines a receiving cavity 121. The rotor 600 and the impeller are located in the receiving cavity 121, with the rotor 600 fixed to the impeller. The rotor 600 has a substantially ring shape. The pump case 120 is provided with a liquid inlet 122 and a liquid outlet 123, and the liquid inlet 122 and the liquid outlet 123 each are in communication with the receiving cavity 121. When energized, the stator assembly 400 generates a magnetic field and drives the rotor 600 to rotate in a suspended state in the receiving cavity 121, thereby causing the impeller to rotate in a suspended state along with the rotor 600 in the receiving cavity 121. The rotating impeller creates a pumping force, allowing liquid to enter the receiving cavity 121 from the liquid inlet 122 and exit the ventricular assist device 10 through the liquid outlet 123, thereby enabling the ventricular assist device 10 to pump liquid.

Referring to FIGS. 2, 4, and 5, the housing 200 includes a bottom wall 201, a cover plate 204, and a peripheral wall 202. The bottom wall 201 and the cover plate 204 are provided opposite each other, and the peripheral wall 202 is connected between the bottom wall 201 and the cover plate 204. The bottom wall 201 and the peripheral wall 202 define an accommodating cavity 210 with an opening, while the cover plate 204 covers the peripheral wall 202 and shields the accommodating cavity 210. The sensing assembly 300, the stator assembly 400, and the control assembly 500 are all located in the accommodating cavity 210. In the present application, the motor 11 is applied to the ventricular assist device 10, and the pump case 120 covers the opening of the accommodating cavity 210, that is, the cover plate 204 is a part of the pump case 120. Specifically, the cover plate 204 serves as the bottom cavity wall of the receiving cavity 121. In other embodiments, the motor 11 can also be applied to other devices, in which case the cover plate 204 can only be a part of the housing 200 of the motor 11. In the present application, the housing 200 is substantially in a volute shape, causing the accommodating cavity 210 defined by the housing 200 to be substantially in a volute shape.

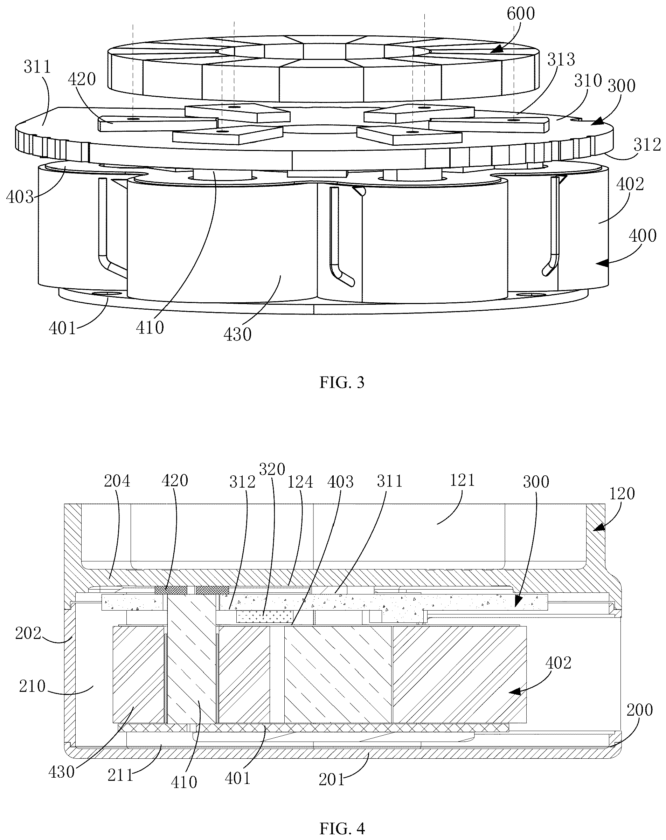

Referring to FIGS. 3 to 5, in some embodiments, the bottom wall 201 has a bottom wall surface 211 that defines a part of the boundary of the accommodating cavity 210. The bottom wall surface 211 of the housing 200 can be understood as the bottom cavity surface of the accommodating cavity 210. The pump case 120 also has a top wall surface 124, which is provided opposite the bottom wall surface 211. The top wall surface 124 can be understood as the top cavity surface of the accommodating cavity 210.

The stator assembly 400 is accommodated within the accommodating cavity 210. The stator assembly 400 includes a back plate 401, a winding unit 402, and an insulating film 403. A plurality of winding units 402 are provided. The plurality of winding units 402 are arranged on the back plate 401. For example, the plurality of winding units 402 are arranged in a circular array on the back plate 401. Each winding unit 402 includes a magnetic core 410, a pole shoe 420, and a coil 430.

The magnetic cores 410 of the plurality of winding units 402 are arranged in a circle, and extension directions of the plurality of winding units 402 are parallel. The extension direction of each magnetic core 410 is parallel to the axial direction of the stator assembly 400. The rotor 600 is provided on the side of the cover plate 204 away from the stator assembly 400. Specifically, the rotor 600 is provided in the receiving cavity 121 defined by the pump case 120. The magnetic core 410 is cylindrical in shape, and the central axis of the magnetic core 410 extends through the rotor 600, so that the magnetic core 410 is approximately aligned with the rotor 600. That is, the region of the rotor 600 with a relatively high magnetic field intensity is approximately aligned with the magnetic core 410, thereby increasing the reaction force exerted by the rotor 600 on the stator assembly 400, increasing the action force exerted by the stator assembly 400 on the rotor 600, and ultimately enhancing the driving efficiency of the stator assembly 400 on the rotor 600. The central axis of the magnetic core 410 refers to the central axis of the column where the magnetic core 410 is located. One end of the magnetic core 410 is fixed to the back plate 401, and the other end thereof is fixed to the pole shoe 420.

The side of the pole shoe 420 away from the magnetic core 410 is in contact with the cover plate 204, that is, the pole shoe 420 is in contact with the top wall surface 124. As such, the top wall surface 124 can position the pole shoe 420, thus facilitating the correct assembly of the pole shoe 420 and the magnetic core 410. Moreover, the heat generated by the magnetic core 410 and pole shoe 420 during operation can be transferred to the external environment through the pump case 120, improving the heat dissipation effect of the motor 11 and the ventricular assist device 10. Additionally, the pole shoe 420 is closer to the impeller, enhancing the driving efficiency of the stator assembly 400.

The coil 430 is sleeved on the magnetic core 410. When energized, the coil 430 can generate a rotating magnetic field to drive the impeller to rotate.

The insulating film 403 is provided between the coil 430 and the sensing assembly 300. The insulating film 403 has excellent insulation performance.

Referring to FIGS 4 to 6, the sensing assembly 300 includes a sensing circuit board 310, which is accommodated in the accommodating cavity 210. The stator assembly 400 extends through the sensing circuit board 310. Specifically, the sensing circuit board 310 is provided substantially perpendicular to the axial direction of the stator assembly 400 in the accommodating cavity 210. The sensing circuit board 310 is more adjacent to the top wall surface 124 of the pump case 120 than the back plate 401. That is, when the ventricular assist device 10 is placed upright (with the liquid inlet 122 facing upward), the sensing circuit board 310 is located above the back plate 401. The sensing circuit board 310 has opposing upper surface 311 and lower surface 312, and the upper surface 311 is more adjacent to the top wall surface 124 than the lower surface 312. That is, the upper surface 311 is farther from the bottom wall surface 211 of the accommodating cavity 210 than the lower surface 312, and the lower surface 312 is more adjacent to the bottom wall surface 211 than the upper surface 311.

The sensing circuit board 310 includes a circuit board body 313 and a connecting portion 314. The circuit board body 313 is substantially disc-shaped, and the connecting portion 314 protrudes from the peripheral side of the circuit board body 313. The connecting portion 314 is substantially triangular in shape and is configured to mount the controlling chip of the control assembly 500. In the present embodiment, the circuit board body 313 and the connecting portion 314 are integrally formed. In other embodiments, the circuit board body 313 and the connecting portion 314 can also be connected by bonding or welding. The magnetic core 410 extends through the circuit board body 313 of the sensing circuit board 310. Since the pole shoe 420 is in contact with the top wall surface 124 of the pump case 120, the pole shoe 420 is located between the top wall surface 124 and the circuit board body 313. The connecting portion 314 is electrically connected to the control assembly 500.

Referring to FIG. 5, since the accommodating cavity 210 is substantially volute-shaped, with a circular region and a substantially triangular region, and conventional stators are substantially cylindrical in shape, it is difficult to fully utilize the space of the volute-shaped accommodating cavity 210. In the ventricular assist device 10 of the present application, the sensing circuit board 310 is configured with a circuit board body 313 and a connecting portion 314 protruding from the peripheral side of the circuit board body 313, so that an outer contour of the sensing circuit board 310 is more closely attached to the volute-shaped accommodating cavity 210, enabling connecting portion 314 and the controlling chip mounted thereon to make full use of the existing space of the accommodating cavity 210. The existing space refers to the substantially triangular region of the volute shape. There is no need to additionally provide a circuit board for mounting the controlling chip, which avoids occupying axial space of the motor 11, reduces the thickness of the motor 11, and consequently decreases the thickness of the ventricular assist device 10.

Referring to FIGS. 3 to 5, in some embodiments, an end of the magnetic core 410 away from the back plate 401 is coplanar with an upper surface 311 of the circuit board body 313, and the pole shoe 420 is fixed to the end of the magnetic core 410 away from the back plate 401. The fixing method can be welding or bonding, and the pole shoe 420 extends in the radial direction of the magnetic core 410, so that the pole shoe 420 fixed to the upper end of the magnetic core 410 can be in contact with the upper surface 311 of the circuit board body 313. The pole shoe 420 is provided between and in contact with the upper surface 311 of the circuit board body 313 and the top wall surface 124 of the pump case 120, so that the gap between the upper surface 311 and the top wall surface 124 is equal to the thickness of the pole shoe 420, which minimizes the gap between the upper surface 311 and the top wall surface 124, thereby reducing the thickness of the motor 11.

The sensing assembly 300 further includes a sensor 320 electrically connected to the sensing circuit board 310. The sensor 320 is provided on a lower surface 312 of the circuit board body 313, i.e., the sensor 320 is in physical contact with the side of the circuit board body 313 away from the pole shoe 420. The sensor 320 can detect the rotational speeds and levitation height of the rotor 600 and the impeller. The sensor 320 can transmit the detected information to the control assembly 500 via the sensing circuit board 310. The control assembly 500 can adjust the current of the coil 430 based on the detected information, thereby regulating and controlling the rotational speeds and levitation height of the rotor 600 and the impeller.

In some embodiments, the thickness of the sensor 320 is greater than that of the pole shoe 420. The thickness of the sensor 320 is substantially fixed due to limitations of its internal chip structure. For example, the thickness of the sensor 320 can be about 0.75 mm. The thickness of the pole shoe 420 can be in a range from 0.3 mm to 0.5 mm, for example, can be 0.3 mm, 0.4 mm, 0.5 mm, etc., ensuring that the pole shoe 420 cannot deform without increasing the thickness of the motor 11. The pole shoe 420 that is too thin can lead to deformation, while the pole shoe 420 that is too thick can impact the overall thickness of the motor 11 and the ventricular assist device 10.

Stable operation of the ventricular assist device 10 requires to satisfy at least two following conditions: First, the pole shoe 420 requires to be in contact with the top wall surface 124 of the pump case 120, which allows the top wall surface 124 to position the pole shoe 420, enables heat generated by the coil 430 to transfer to the external environment through the pump case 120, and also enables the pole shoe 420 to be more adjacent to the impeller, improving the driving efficiency of the stator assembly 400. Second, to ensure detection accuracy of the sensor 320, it is necessary to maintain a certain axial spacing between the sensor 320 and the top wall surface 124. If the axial spacing between the sensor 320 and the top wall surface 124 is too small, the magnetic flux received by the sensor 320 is too large, causing the sensor 320 to experience magnetic flux saturation, which can lead to distortion and reduced detection accuracy. If the axial spacing between the sensor 320 and the top wall surface 124 is too large, the magnetic flux received by the sensor 320 is insufficient, which can also degrade detection accuracy.

As described above, when the upper end of the magnetic core 410 is coplanar with the upper surface 311 of the circuit board body 313, the pole shoe 420 fixed to the upper end of the magnetic core 410 will be in contact with the upper surface 311 of the circuit board body 313. Since the sensor 320 is thicker than the pole shoe 420 and a certain axial spacing between the sensor 320 and the top wall surface 124 is required, if the sensor 320 is provided on the upper surface 311 of the circuit board body 313, the pole shoe 420 cannot be in contact with the top wall surface 124. In this case, to ensure the contact between the pole shoe 420 and the top wall surface 124, the length of the magnetic core 410 should be increased to ensure the upper end of the magnetic core 410 being located above the upper surface 311, thereby making the pole shoe 420 fixed to the upper end of the magnetic core 410 to satisfy the condition of contact with the top wall surface 124. As such, the length of the magnetic core 410 can be increased. When the sensing assembly 300 and the stator assembly 400 are considered as a whole, the increase in the overall axial length can result in an increased axial spacing between the top wall surface 124 and the bottom wall surface 211, that is, an increased axial length of the accommodating cavity 210, which leads to an increase in the thickness of the motor 11 and the ventricular assist device, causing the ventricular assist device 10 to be prone to being in contact with other thoracic organs and thereby compromising its safety during use.

In the present application, by providing the sensor 320 on the lower surface 312 of the circuit board body 313, the length of the magnetic core 410 can be reduced, thereby decreasing the axial thickness of the motor 11 and the ventricular assist device 10. As a result, the risk that the ventricular assist device 10 may be in contact with other thoracic organs when implanted can be reduced, and the safety of the ventricular assist device 10 can be improved. Additionally, by providing the sensor 320 on the lower surface 312 of the circuit board body 313, the sensor 320 is farther from the top wall surface 124 compared to the solutions in which the sensor 320 is provided on the upper surface 312 of the circuit board body 313. The spacing between the sensor 320 and the top wall surface 124 is substantially equal to the sum of the thicknesses of both the pole shoe 420 and the circuit board body 313, thereby ensuring that the spacing between the sensor 320 and the top wall surface 124 meets the requirements for detection accuracy.

Referring to FIGS. 4, 5, and 7, the sensor 320 is provided on the lower surface 312 of the circuit board body 313, and is located between the circuit board body 313 and the coil 430. In order to prevent contact between the coil 430 and the sensor 320, the axial length of the coil 430 requires to be reduced. However, in order to maintain the magnetic field strength when the coil 430 is energized, the number of coil turns remain unchanged. Thus, the diameter of individual coil wires can be appropriately reduced to ensure the magnetic field intensity.

The insulating film 403 is provided between the coil 430 and the sensing assembly 300, specifically between the coil 430 and the sensor 320. The outer contour of the insulating film 403 is substantially matched with the outer contour formed by a plurality of coils 430, so as to cover the ends of the plurality of coils 430, thereby further isolating the sensor 320 from the coils 430. The insulating film 403 is provided with a through-hole allowing the magnetic core 410 to extend pass. The isolation and insulation effect of the insulating film 403 can prevent the sensor 320 from being in contact with the coil 430 to form an electrical conduction relationship. As such, the heat of the coil 430 can be prevented from being transferring to the sensor 320 to damage the sensor 320. Moreover, the instantaneous high-voltage of the coil 430 can be prevented from damaging the sensor 320 and other components on the sensing circuit board 310. A gap can also form between the sensor 320 and the insulating film 403, so that the sensor 320 and the insulating film 403 maintain a non-contact relationship, which is more conducive to preventing the sensor 320 from being in contact with the coil 430 to form an electrical conduction relationship.

Referring to FIGS. 2 and 5, in some embodiments, a plurality of sensors 320 and a plurality of magnetic cores 410 are provided. Each sensor 320 is located between adjacent two magnetic cores 410. The plurality of sensors 320 are arranged in a circular array along the central axis of the stator assembly 400.

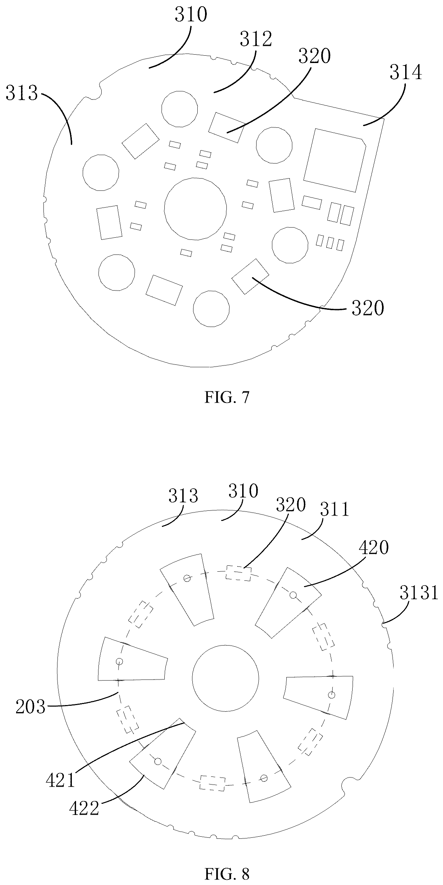

As shown in FIGS. 7 and 8, the sensor 320 is substantially cuboid in shape and has a width direction and a length direction. In the present application, the width direction of each sensor 320 is consistent with the radial direction of the stator assembly 400, and the length direction of the sensor 320 is substantially tangential to the circular array formed by the plurality of sensors 320, so that the outer and inner contours of the plurality of sensors 320 can form circles, and the plurality of sensors 320 as a whole substantially constitute a ring. Compared to an arrangement where the length direction of the sensor 320 is consistent with the radial direction of the stator assembly 400, the ring formed by the plurality of sensors 320 in the present application has a smaller width, thus allowing the orthographic projection of the sensors 320 onto the rotor 600 to overlap with the rotor 600 as much as possible, which improves the accuracy of the detected information, e.g., rotational speeds and levitation height of the rotor 600 and impeller that can be detected by the sensor 320 and enhances the detection accuracy of the sensor 320.

Referring to FIG. 8, the central axes of the plurality of the magnetic cores 410 form a cylindrical surface 203. That is, the central axes of the plurality of magnetic cores 410 all lie on the cylindrical surface 203. Since the coils 430 are sleeved on the magnetic cores 410, the central axis of each magnetic core 410 is also the central axis of its corresponding coil 430. In the illustrated embodiment, the center of each sensor 320 is located on the cylindrical surface 203. This means the projection of the sensor 320 on the sensing circuit board 310 and the projection of the magnetic core 410 on the sensing circuit board 310 lie on the same circumference. Since the central axis of the magnetic core 410 extends through the rotor 600, the magnetic core 410 is axially aligned with the rotor 600, the sensor 320 is also axially aligned with the rotor 600, thus further improving detection accuracy of the sensor 320.

Referring to FIGS. 5 and 8, in some embodiments, the width of the pole shoe 420 gradually increases along the direction from the center of the circuit board body 313 to the edge of the circuit board body 313, so that the pole shoe 420 has a substantially trapezoidal shape. As such, the magnetic field generated by the stator assembly 400 exhibits an excellent linear distribution. The pole shoe 420 has a first end 421 and a second end 422 along the radial direction of the stator assembly 400. The first end 421 is more adjacent to the center of the sensing circuit board 310 than the second end 422, i.e., the second end 422 is more adjacent to the edge of the circuit board body 313. The width of the first end 421 is less than that of the second end 422.

The first end 421 and the second end 422 are located on the inner and outer sides of the cylindrical surface 203. The distance from the first end 421 to the cylindrical surface 203 is greater than the distance from the second end 422 to the cylindrical surface 203. Compared to an arrangement where the distance from the first end 421 to the cylindrical surface 203 is equal to the distance from the second end 422 to the cylindrical surface 203, the foregoing arrangement makes the radius of the circle corresponding to the cylindrical surface 203 larger, thereby enlarging the radius of the coil 430 and increasing the number of coil turns. Consequently, the driving efficiency of the motor 11 is higher.

Referring to FIGS. 5, 9, and 10, in some embodiments, the control assembly 500 includes a fixing member 510 and a control circuit board 520. The fixing member 510 is mounted in the accommodating cavity 210, and the control circuit board 520 is mounted on the fixing member 510. The control circuit board 520 is electrically connected to the sensing circuit board 310.

Specifically, the fixing member 510 is provided between the connecting portion 314 and the inner wall of the housing 200. In the illustrated embodiment, one fixing member 510 is provided. The contour of the side of the fixing member 510 facing the connecting portion 314 corresponds to the contour of the connecting portion 314. That is, the contour of the side of the fixing member 510 facing the connecting portion 314 is substantially the same as the contour of the connecting portion 314, for example, both are L-shaped, arc-shaped, or another shape, so that the fixing member 510 can be as close as possible to the connecting portion 314, minimizing the gap between the fixing member 510 and the connecting portion 314, thereby reducing the volume of the motor 11 and the ventricular assist device 10.

In this embodiment, the contour of the side of the fixing member 510 away from the connecting portion 314 corresponds to a part of the contour of the inner wall of the housing 200. That is, the contour of the side of the fixing member 510 away from the connecting portion 314 is substantially the same as a part of the contour of the inner wall of the housing 200, so that the fixing member 510 can make full use of the space within the accommodating cavity 210, thereby reducing the volume of the motor 11 and the ventricular assist device 10.

A plurality of control circuit boards 520 can be provided and are electrically connected to each other, and one of the control circuit boards 520 is electrically connected to the sensing circuit board 310. The fixing member 510 is provided with a plurality of mounting slots 511 and at least one wire slot 512. The plurality of mounting slots 511 accommodate the plurality of the control circuit boards 520, respectively. Each control circuit board 520 is accommodated in one slot mounting slot 511. Adjacent two mounting slots 511 are in communication with each other by a wire slot 512, and each wire slot 512 can hold the wire 513 connecting adjacent two control circuit boards 520. By providing the wire slot 512 on the fixing member 510, not only the wire 513 can be held to facilitate the assembly of the control assembly 500, but also the space occupied by the wire 513 can be saved, contributing to the miniaturization of the motor 11 and the ventricular assist device 10.

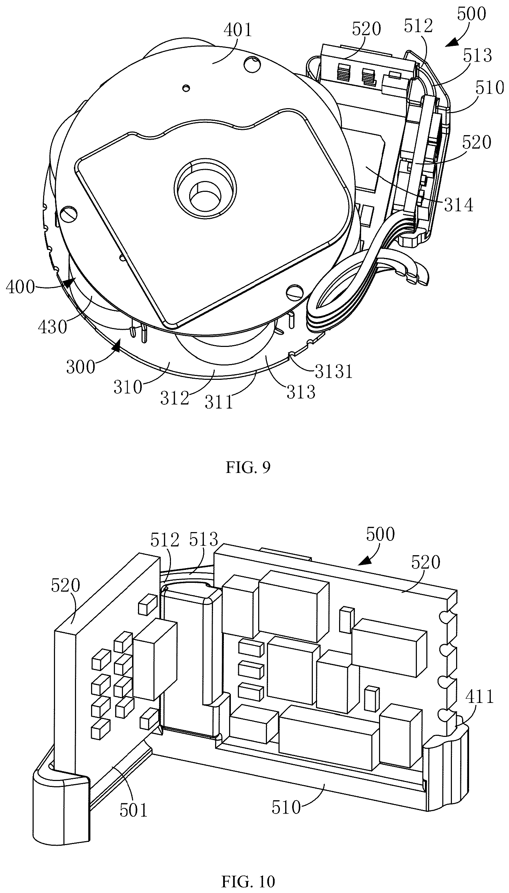

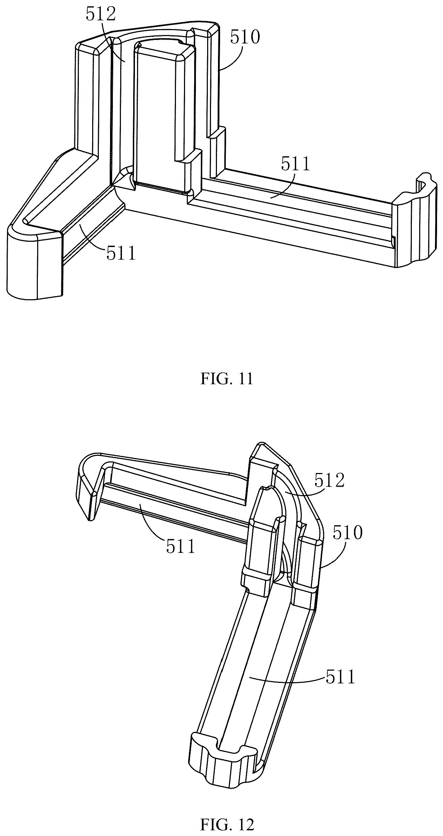

Referring to FIGS. 10 to 12, the area of a single control circuit board 520 is less than the area of the circuit board body 313. In other words, when a single control circuit board 520 and the circuit board body 313 are placed horizontally, the coverage area of the circuit board body 313 is greater than that of the single control circuit board 520. The control circuit board 520 is provided on the peripheral side of the stator assembly 400, specifically, between the stator assembly 400 and the peripheral wall 202. When the ventricular assist device 10 is fully assembled and placed upright, the circuit board body 313 remains horizontal, while the control circuit board 520 is provided vertically, i.e., the control circuit board 520 is substantially provided along the axial direction of the stator assembly 400. The control circuit board 520 has an upper end surface 501 provided away from the bottom wall surface 211 of the accommodating cavity 210. The side of the control circuit board 520 away from the bottom wall 201 does not exceed the surface of the sensing circuit board 310 away from the bottom wall 201. The distance from the upper end surface 501 of the control circuit board 520 to the bottom wall surface 211 is less than or equal to the distance from the upper surface 311 to the bottom wall surface 211. In other words, the height of the upper end surface 501 of the control circuit board 520 relative to the bottom wall surface 211 does not exceed the height of the upper surface 311 relative to the bottom wall surface 211, thus preventing an increase in the thickness of the motor 11.

If the sensing circuit board 310 is provided adjacent to an axial end of the stator assembly 400, and the control circuit board 520 is provided adjacent to the other axial end of the stator assembly 400, the sensing circuit board 310, the stator assembly 400, and the control circuit board 520 are arranged sequentially along the axial direction. When the control circuit board 520, the stator assembly 400, and the sensing circuit board 310 are regarded as an integral assembly, the axial length of the integral assembly is relatively large, so that the axial length (i.e., thickness) of the motor 11 and the ventricular assembly device 10 including the motor 11 is relatively large. When a ventricular assist device 10 having a relatively large thickness is implanted into a patient, the risk of contact between the ventricular assist device 10 and other organs in the thoracic cavity can be increased, thereby improving the safety of use of the ventricular assist device 10.

As for the motor 11 and ventricular assist device 10 of the present embodiment, the plurality of control circuit boards 520 are provided on the peripheral side of the stator assembly 400, and the height of the control circuit boards 520 in the thickness direction of the sensing circuit board 310 is lower than that of the sensing circuit board 310. Compared to the arrangement of the control circuit boards 520 in the axial direction of the stator assembly 400, this arrangement reduces the space required for installation of the control circuit boards 520 in the axial direction, thereby decreasing the thickness of the motor 11 and the ventricular assist device 10, which significantly reduces the risk of contact between the ventricular assist device 10 and other organs in the thoracic cavity, thereby improving the safety of use of the ventricular assist device 10.

In some embodiments, the fixing member 510 has a top end away from the bottom wall surface 211 of the accommodating cavity 210. The distance from the top end of the fixing member 510 to the bottom wall surface 211 is less than or equal to the distance from the upper surface 311 to the bottom wall surface 211. That is, the fixing member 510 is also lower than the sensing circuit board 310 in the thickness direction of the sensing circuit board 310, ensuring that the entire control assembly 500 does not exceed the sensing circuit board 310, which is conducive to reducing the thickness of the motor 11 and the ventricular assist device 10. The fixing member 510 is made of metal and is in contact with the housing 200, so that the fixing member 510 exhibits excellent thermal conductivity effect, allowing heat generated during the operation of the control circuit board 520 to be quickly conducted through the fixing member 510 to the housing 200 and then dissipated to the external environment, thereby improving the heat dissipation effect of the motor 11 and the ventricular assist device 10.

In some embodiments, the control circuit board 520 is provided parallel to the axial direction of the stator assembly 400, while the sensing circuit board 310 is perpendicular to the axial direction of the stator assembly 400. Therefore, the control circuit board 520 is perpendicular to the sensing circuit board 310. When a plurality of control circuit boards 520 are provided, each control circuit board 520 is parallel to the axial direction of the stator assembly 400. By providing the control circuit board 520 between the stator assembly 400 and the peripheral wall 202 of the housing 200 in parallel to the axial direction of the stator assembly 400 without inclining the control circuit board 520 with respect to the stator assembly 400, the radial space occupied by a single control circuit board 520 is minimized while reducing the axial thickness of the motor 11, thereby facilitating the miniaturization of the motor 11 and the ventricular assist device 10.

In summary, the control circuit board 520 of the motor 11 according to the present application is provided on the peripheral side of the stator assembly 400. Compared to the arrangement of the control circuit boards 520 in the axial direction of the stator assembly 400, this arrangement reduces the space required for installation of the control circuit boards 520 in the axial direction, thereby decreasing the thickness of the motor 11 and the ventricular assist device 10, which significantly reduces the risk of contact between the ventricular assist device 10 and other organs in the thoracic cavity, thereby improving the safety of use of the ventricular assist device 10.

The above embodiments are only used to illustrate the technical solutions of the present application and are not intended to limit the present application. Although the present application has been described in detail with reference to the foregoing embodiments, those of ordinary skill in the art should understand that the technical solutions described in the foregoing embodiments can still be modified, or some of the technical features can be equivalently replaced. These modifications or replacements do not cause the essence of the corresponding technical solutions to deviate from the spirit and scope of the technical solutions of the embodiments of the present application, and should be included within the protection scope of the present application.

## Claims

1. A motor, comprising:
a housing defining an accommodating cavity;
a sensing assembly comprising a sensing circuit board accommodated in the accommodating cavity;
a stator assembly extending through the sensing circuit board; and
a control assembly comprising a control circuit board electrically connected to the sensing circuit board, wherein the control circuit board is accommodated in the accommodating cavity and is provided at a peripheral side of the stator assembly.

2. The motor according to claim 1, wherein the housing comprises a bottom wall and a peripheral wall connected to the bottom wall, the bottom wall and the peripheral wall define the accommodating cavity, and the control circuit board is provided between the stator assembly and the peripheral wall.

3. The motor according to claim 2, wherein a side of the control circuit board away from the bottom wall does not exceed a surface of the sensing circuit board away from the bottom wall.

4. The motor according to claim 1, wherein the sensing circuit board comprises a circuit board body and a connecting portion protruding from a peripheral side of the circuit board body, and the connecting portion is electrically connected to the circuit board body and the control circuit board.

5. The motor according to claim 1, wherein the control circuit board is perpendicular to the sensing circuit board, and the sensing circuit board is perpendicular to an axial direction of the stator assembly.

6. The motor according to claim 1, wherein a plurality of the control circuit board are provided, the plurality of control circuit boards are electrically connected to each other, one of the plurality of the control circuit boards is electrically connected to the sensing circuit board; the control assembly further comprises a fixing member fixedly provided in the accommodating cavity, the fixing member is provided with a plurality of mounting slots and at least one wire slot, the plurality of mounting slots is configured to accommodate the plurality of control circuit boards, each control circuit board is accommodated in one mounting slot, adjacent two mounting slots are in communication with each other through one wire slot, and each wire slot is configured to accommodate a wire connecting adjacent two control circuit boards.

7. The motor according to claim 6, wherein the fixing member is made of metal, and the fixing member is in contact with the housing.

8. The motor according to claim 6, wherein the sensing circuit board comprises a circuit board body and a connecting portion protruding from a peripheral side of the circuit board body, the fixing member is provided between the connecting portion and an inner wall of the housing, and a contour of a side of the fixing member facing the connecting portion corresponds to a contour of the connecting portion.

9. The motor according to claim 8, wherein a contour of a side of the fixing member away from the connecting portion corresponds to a part of a contour of the inner wall of the housing.

10. The motor according to claim 6, wherein the housing comprises a bottom wall and a peripheral wall connected to the bottom wall, the bottom wall and the peripheral wall define the accommodating cavity, the control circuit board is provided between the stator assembly and the peripheral wall; the sensing circuit board has an upper surface and a lower surface opposite each other, the upper surface is farther from the bottom wall of the accommodating cavity than the lower surface, the fixing member has a top end away from the bottom wall, and a distance from the top end of the fixing member to the bottom wall is less than or equal to a distance from the upper surface to the bottom wall.

11. The motor according to claim 1, wherein the housing comprises a bottom wall, a cover plate, and a peripheral wall connected between the bottom wall and the cover plate, the bottom wall and the peripheral wall define the accommodating cavity, the cover plate covers the peripheral wall and shields the accommodating cavity; the stator assembly comprises a magnetic core and a pole shoe, the magnetic core extends through the sensing circuit board, the pole shoe is connected to an end of the magnetic core and is capable of being in contact with the cover plate; the sensing assembly further comprises a sensor electrically connected to the sensing circuit board, and the sensor is provided on a side of the sensing circuit board away from the pole shoe.

12. The motor according to claim 11, wherein the pole shoe is in contact with the sensing circuit board.

13. The motor according to claim 11, wherein the magnetic core is cylindrical in shape, a plurality of magnetic cores are provided, central axes of the plurality of magnetic cores form a cylindrical surface, and a center of the sensor is located on the cylindrical surface.

14. The motor according to claim 13, wherein the pole shoe has a first end and a second end in a radial direction, the first end is more adjacent to a center of the sensing circuit board than the second end, the first end and the second end are located on an inner side and an outer side of the cylindrical surface, respectively, and a distance from the first end to the cylindrical surface is greater than a distance from the second end to the cylindrical surface.

15. The motor according to claim 11, further comprising a rotor provided on a side of the cover plate away from the stator assembly, wherein the stator assembly cooperates with the rotor to drive the rotor to rotate, and a central axis of the magnetic core extends through the rotor.

16. The motor according to claim 11, wherein a plurality of sensors and a plurality of magnetic cores are provided, each sensor is located between adjacent two magnetic cores, the plurality of sensors are arranged in a circular array around a central axis of the stator assembly, each sensor has a width direction, and the width direction of each sensor is consistent with a radial direction of the stator assembly.

17. The motor according to claim 11, wherein a thickness of the pole shoe is less than a thickness of the sensor.

18. The motor according to claim 1, wherein the stator assembly comprises a back plate, a winding unit, and an insulating film, the winding unit is provided on the back plate, the winding unit comprises a magnetic core, a pole shoe, and a coil, an end of the magnetic core is fixed to the back plate, another end of the magnetic core is fixedly connected to the pole shoe, the coil is sleeved on the magnetic core, and the insulating film is provided between the coil and the sensing assembly.

19. The motor according to claim 18, wherein a plurality of winding units are provided, and an outer contour of the insulating film is matched with an outer contour formed by the plurality of coils.

20. A ventricular assist device, comprising the motor according to any one of claims 1 to 19.
